# EUROPEAN PATENT SPECIFICATION

(11) **EP 2 740 539 B1**
(45) Date of publication and mention of the grant of the patent: **13.02.2019**
(21) Application number: 13466030.7
(22) Date of filing: 22.11.2013
(51) Int. Cl.: B04B 1/20, B04B 5/10, C02F 1/38, C02F 11/04, C02F 11/12, C12M 1/107, C12M 1/00, C12M 1/33

(54) **Apparatus for mechanical lysation of a biological sludge thickened in centrifuge**
Vorrichtung zur mechanischen Lysierung von biologischem Schlamm eingedickt in einer Zentrifuge
Appareil pour lyse mécanique d'une boue biologique épaissie dans une centrifugeuse

(30) Priority: 04.12.2012 CZ 20120869
(43) Date of publication of application: 11.06.2014
(73) Proprietor: Kutil, Josef, 290 01 Podebrady (CZ); Dohanyos, Michal, 163 00 Prague 6 (CZ); Zabranska, Jana, 16000 Prague 6 (CZ); Ondra, Tomas, 290 01 Podebrady (CZ)
(72) Inventor: Kutil, Josef, 290 01 Podebrady (CZ); Dohanyos, Michal, 163 00 Prague 6 (CZ); Zabranska, Jana, 16000 Prague 6 (CZ); Ondra, Tomas, 290 01 Podebrady (CZ)
(74) Representative: Beranova, Ivana

(56) References cited:
- EP-A1- 0 807 089
- DE-A1- 19 948 115

## Description

### Field of the Invention

This invention relates to a centrifuge comprising an equipment for mechanical lyzing of the biological sludge thickened in that centrifuge allowing deepening of the organic compounds decomposition in a thickened biological sludge during its anaerobic stabilization with simultaneous decreasing of its weight and increasing of the produced biogas quantity, as well as decreasing of the thickened sludge viscosity, what increases its pumpability when it is thickened up to the double concentration of the dry matter.

### Background of the Invention

The thickened biological sludge, resulting from sewage water treatment by activation, is subjected, in mixture with the primary sludge resulting from sedimentation of the sewage water flowing into the sewage disposal plant, to anaerobic fermentation, from which biogas, containing methane usable for energy production, arises by decomposition of organic compounds in the sludge mixture, .

This state of the art is disclosed for example in patents CZ 291 371, DE 195 02 856 C2, DE 195 02 856 A1, and EP 0 807 089 B1.

The process of anaerobic decomposition of organic compounds can be stimulated by addition of biomass, which biomass was treated by disintegration process, when a part of microbial cells is destroyed and their content - the lysate, containing subcellular components, what are biochemically active compounds, is released into the surrounding atmosphere. The lysate significantly positively co-affects the deeper decomposition of organic compounds in the anaerobic process Therefore, it is the reason of a higher production of the biogas. This method is disclosed in patent CZ 242 979.

Destruction of microbial cells is carried out directly and continually in thickening centrifuges, when a stream of sludge is subjected to destruction in an additional equipment. By mechanical destruction certain part of cells is destructed and a certain amount of lysate is released. This state of art is disclosed in patents CZ 291 371, DE 195 27 784, and EP 0 807 089 B1.

The thickening proper is a common process in the sewage disposal plants, which process is carried out in processing of the activated sludge surplus. The sludge having dry matter concentration from 0.5% by weight to 1.2% by weight is pumped into a centrifuge, and the thickened sludge having concentration 5% by weight to 8% by weight exits the centrifuge. Its high viscosity is at the limit for its pumpability. The thickening is carried out during rotation of the centrifuge drum at from 1800 rpm to 3200 rpm, what depends on the centrifuge size. On the internal surface of the rotating centrifuge drum the thickened sludge is accumulated and the clear liquid, the fugate, is taken away in the centre of the drum. Transport of the thickened sludge is provided by a screw conveyer, the number rotations of which is by several units higher than the number of rotations of the drum. This difference in the number of rotations is the so called rotation differential and it is controllable, because the screw drive is operating independently. By the differential rotations the degree of thickening is controlled, it is the dry matter concentration in the thickened sludge. By increasing the dry matter concentration, the volume of the thickened sludge is decreased, but its viscosity is increased. On the contrary, the volume of the thickened sludge is increased by decreasing of the dry matter concentration and its viscosity is decreased. Viscosity is an important parameter, because its value at a given dry matter concentration is the limit for pumpability of the thickened sludge. Because of this reason, the maximum thickening can reach the value from 6% by weight to 7% by weight, what depends on the delivery pipeline length.

The above-described thickening process is the standard operation of the thickening centrifuges. The pieces of equipment, causing lyzing of the thickened sludge, are situated off the centrifuge drum, and do not have any influence on the thickening process, but on the contrary, the thickening process, it is the concentration and the volume of the thickened sludge, have influence on the efficacy of the lyzing in the additional equipment for mechanical lyzing of the thickened sludge which left the centrifuge drum.

The additional lyzing equipment must not be an impediment to the continual thickening process, must not have any negative influence on the separation effect of the centrifuge, and in any case it must not form such obstruction for the stream of the thickened sludge at the output from the centrifuge drum, that would cause its congestion, i.e. interruption of its operation and a shut-down with the following disassembly, cleaning, checking of the equipment state, assembly and putting into operation again.

The known lyzing equipment designs are disclosed in patents CZ 291 371, DE 195 27 784, and EP 0 807 089 B1.

A friction grinding machine with grinding disks, where one of them is stable and the other one is joined with the rotating centrifuge drum, is described, and destruction of the microbial cells in the sludge is caused by its mechanical grinding between the discs having coarse surface. The gap between both discs is controllable from 0.5 mm to 5 mm. Disadvantages of such design consist first of all in that the gap between the discs, even if controllable, is an obstacle for free flow of the thickened sludge out of the centrifuge and often causes its congestion. But the gap width is decisive for efficacy of the lyzing. The extent of destruction of the microbial cells increases with the decreasing of the gap. However, this decreasing causes congestion of the centrifuge, what is considered as an operational failure. Increasing of the gap makes output of the thickened sludge easier, but the lyzing efficiency rapidly decreases in case of this design. The equipment is a part of the output end of the centrifuge. Therefore, any manipulation with the equipment, e.g. adjusting of the gap between the discs, requires its shut-down. Surface of the grinding discs can be damaged by solid particles, which particles can enter the equipment together with the thickened sludge. Therefore, a sieve is used. However, such sieve is difficult to place in the structure, and moreover, it represents another hydraulic resistance hindering free flow of the thickened sludge from the centrifuge, and so it contributes to its congestion. From time to time, this sieve has to be cleaned, what requires a shut-down of the equipment.

Another described friction grinding machine comprises grinding cones, of which one is stable and the other is joined with the rotating centrifuge drum. Width of the gap can be adjustable. Disadvantages of this design are identical to those of the equipment with the grinding discs. Therefore, their description is identical as the description of the previous design.

A profiled grater is described, where the friction surfaces of the stationary casing and of the rotation friction surface joined with the centrifuge drum are situated axially. A narrow gap having width from 1 mm to 10 mm is situated between the friction surfaces, which gap is narrowing towards both sides in the direction to the sledge output. A disadvantage of this design consists in the congestion of the centrifuge, because to reach the lyzing effect, the gap has to be adjusted to be as narrow as possible, i.e. nearly equal to 1 mm, but because of this reason, the free flow of the thickened sludge downstream of the centrifuge is limited. If such gap is selected to prevent congestion, it rapidly decreases the effect of lyzing by pressing and wiping. Also, it is necessary to install a sieve to prevent entering of solid objects, what also restrains free flow of the sludge from the z centrifuge and contributes to its congestion. However, the possibility to adjust the gap requires as necessity to shut-down the centrifuge and to put it out of operation, what is also disadvantageous.

The described roller squeezer is lyzing the thickened sludge by pressure, when it passes through several sets of rollers, wherein one set consists at least of ten rollers. The sludge throughput is controlled by the output opening size. Variability of the lyzing process consists in the possibility to decide about the number of rollers, the number of sets and about the gap between the rollers. The entire equipment has to be manufactured very precisely and it is sensitive to any entering of solid particles in the same way as it is in the case of all above described friction machines. Therefore, a sieve has to be placed in the upstream. Another disadvantage is that the whole system is very complicated and its low throughput for the thickened sludge is a cause of congestion, but the possible control requires again a shut-down of the centrifuge to put it out of operation.

A disadvantage of the described extrusive drum is that its gap is narrow. The gap is the limiting factor of free passage of the sludge from the centrifuge. In case of discrepancy between sludge volume exiting the centrifuge and the flow rate possibilities of the extrusive drum congestion of the centrifuge may happen. In case of the extrusive elements, which are firmly joined with the rotating part of the equipment, destruction takes place very frequently, so that the extrusive elements brake in the place where they are joined with the rotating part. That is, the elements are strained by their movement in the layer of the thickened sludge and also by centrifugal force. The described unit of a cutting machine is lyzing the thickened sludge by means of cutting elements that are arranged as rows of rotating elements that reach into gaps of the static cutting elements. In this effect, the lyzing effect is realized by shear forces. The shear forces are effective when the distance between a static and a rotating cutting elements is minimal, and therefore, the shear effect of edges of both elements can be fully effective. A disadvantage of this design is the large number of cutting elements in many rows, what is an obstacle for free flow of sludge from the centrifuge, and therefore, congestion takes place. The system is very complicated. The gap width has to respect what does the centrifuge drum, with which drum the rotating part of the system is firmly joined. During the course of running the centrifuge drum axially moves up to 6 mm in dependence on the diameter and the length of the centrifuge drum, and this in both directions. This fact caused failures of the cutting units in case that the gap set between the cutting units has been smaller than 5 mm. Adjustability of different gap sizes between the static and rotating cutting elements is complicated and requires design modifications. This is a disadvantage of this equipment. Also, controlling of the output adjuster at the lyzed sludge output is possible only one time, and this after a shut-down and opening of the centrifuge.

A disadvantage of the design of the peg grinding unit is that the lyzing takes place predominantly by impact, and this in a one-way stream of the thickened sludge. Number of the static and rotating pegs and their diameter have to correspond to the amount of the flowing sludge to prevent centrifuge congestion. Adjustability of the pegs is possible only after a centrifuge shut-down, its opening and disassembly of the peg grinding unit by replacement of the cutting elements by such ones that have different layout of the pegs and by repeated assembly, closing of the centrifuge and putting it into operation. Control of the residence time by means of the adjuster also requires a centrifuge shut-down, its opening, modifying of the adjuster position, repeated closing of it and putting it into operation.

A common disadvantage of all the above described designs is that reliable functionality can be reached only in case of a constant sludge inlet into the thickening centrifuge at a constant dry matter concentration in the sludge inlet, and at constant rotation speed difference, when the thickening of sludge is constant, and also the thickened sludge volume, extruded from the centrifuge by screw conveyor, is constant within a time unit. In real operation, this set of conditions cannot be kept in a continual and long-time operation. Another common drawback of all above described designs is that in case of any centrifuge there is an axial shift of the drum in both directions from 1 mm to 6 mm, the cause of which are temperature dilatations.

When there is a change in the dry matter concentration within the thickened sludge, which concentration depends on the dry matter concentration change of the inputting non-thickened sludge, its input volume, and setting of the:rotation speed difference, there is also a change in its volume. For example, if there is any decrease in the dry matter concentration in the thickened sludge from 7% by weight to 5% by weight, the thickened sludge volume is increased by 30%. The variations in volume and density of the sludge cause congestion in the centrifuge because it is not possible to adopt flow rate in the above described systems to such changes during the operation of the centrifuge without their controlling or even without disassembly, modification and adjustment of such elements, the following re-assembly and putting of the centrifuge in operation.

A decrease in the sludge throughput in the known lyzing equipment causes not only centrifuge congestion that is putting it out of operation, but also a rapid surge in the electric power consumption, what can cause an overload of the main motor, its stoppage, and eventually failure. The described equipment has a disadvantage consisting in that it can be adjusted only to a certain operational state of the centrifuge. Any operational state of the centrifuge requires certain modification of the described lyzing equipment, sometimes also a design modification, what is a disadvantage in real operation where the input characteristics of the thickened sludge are modified frequently. Further, such facts either negatively influence operation of the centrifuge (congestion), what is undesired in a continual operation, or the reached lyzing effect is at a low level. The thickened sludge contains solid particles having size up to several millimetres in variable quantities. Such solid objects are to be removed (sieves) to prevent damage of the lyzing equipment, or even of the centrifuge.

### Summary of the invention

Subject matter of this invention is a centrifuge comprising an equipment for mechanical lyzing of biological sludge thickened in that centrifuge, which equipment comprises rotating parts placed on a centrifuge drum and static parts joined firmly with a housing of the centrifuge, the substance of which consists in that the rotating parts are four-square rotating elements symmetrically disposed between output openings of the drum for the thickened sludge, wherein the static part consists of two outside partitions and one central partition in the shape of an annulus, which partitions are arranged on a load-bearing ring in the centrifuge housing so that arms of the four-square rotating elements reach into the gaps between them.

Preferably, a gap is formed in the four-square rotating elements, into which the central partition reaches.

Preferably, the four-square rotating elements are fastened to the centrifuge drum by screws so that a stream of thickened sludge passes through the gap.

Preferably, the outside partitions and the central partition are formed in the shape of a divided annular area, wherein the outside partitions are provided with openings and the central partition is provided with cut-outs.

Substance of this invention is a mechanical disintegrator adapted as an additional equipment to the thickening centrifuge for lyzing of the thickened biological sludge, which additional equipment forms one whole with the centrifuge.

For destruction of the microbial cells to free the cell lyzate the equipment according to this invention uses both the set of the disintegration elements and the kinetic energy of the rotating centrifuge drum and the kinetic energy of the output stream of the thickened sludge.

The equipment for mechanical disintegration of the thickened biological sludge according to the invention removes deficiencies of the mechanical equipment for lyzing known from the prior art, e.g. of the machine cutting unit and the peg grinding unit.

The main advantage of the equipment according to the invention consists in that the lyzing equipment is a part of the thickening centrifuge, and thereby thickening of the biological sludge and its following disintegration, i.e. lyzing, takes place in one machine.

The disintegration equipment according to the invention is different from the already known disintegration equipment assembled on the thickening centrifuges in that has a special, which design is simpler, allows reliable, failure-free operation at varying centrifuge load, does not influence the separation effect of the centrifuge, uses the kinetic energy of the rotating centrifuge drum more effectively , and it is not necessary to adjust it, and to shut down the centrifuge from operation because of this reason, this all at high lyzing efficiency.

The rotating elements of the disintegration equipment according to the invention are incompact, they have small weight, and therefore, they are not destucted by the centrifugal force action. The high throughput of the thickened sludge disintegration equipment according to this invention at providing of a high degree of lyzing by co-action of an impact, shear stress, and cavitation provides its resistance to destruction in case of the possible intrusion of solid particles.

According to the invention, disintegration of the microbial cells is caused by a combination of several factors, and this by mechanical forces - by impact of particles of the thickened sludge on parts of the equipment and further by cavitation and also by action of the shear stress.

The disintegrator consists of rotating elements and static elements. The rotating elements are placed symmetrically on the centrifuge drum so that adding of weight of the fixed rotating elements does not disturb dynamic balance of the centrifuge drum. Their arrangement does not restrict the thickened sludge stream from the centrifuge. Therefore, centrifuge congestion cannot take place in any of its operational modes. The disintegrator rotating elements are fastened directly on the output part of the centrifuge drum, and this on surfaces that are between openings, by which the thickened sludge exits from the drum. The number of rotating elements is selectable, their final number is limited by the number of drum surfaces between the output openings at maintaining the dynamic balance of the drum.

The exiting stream of sludge from the centrifuge without any lyzing treatment is retained by its retaining equipment, suitably formed as a structural part of the centrifuge that is for example by the housing of the terminal cylindrical part of the centrifuge drum, which according to the invention is preferably used after modification for building in of disintegrator static elements. An advantage of the disintegrator according to the invention is that it requires minimal interventions into the modified centrifuge, for installation it uses its structure, and first of all it does not restrict the stream of the thickened sludge, what causes that the disintegrator according to the invention requires minimal interventions into its structure.

The stream of the thickened sludge exits the centrifuge through output openings in the form of a rotating disc, i.e. as rotating mass of the thickened sludge, wherein its plane is perpendicular to the centrifuge axis, and thickness of this rotating mass is 1 - 2 cm.

The disintegrator according to the invention suitably uses this phenomenon. It uses the kinetic energy of this stream by that the inserted central shaped partition, joined with the housing, is placed so that it is situated in the middle of the target stream of sludge, which stream is divided on it, impacts on surfaces of the shaped partition, what causes lyzing by impact, the effect of which lyzing is increased by impacts of the rotating elements surfaces, which surfaces are moving at both sides of the central partition. In the gaps on both sides of the partition impact of the working surfaces of the rotating elements on sludge stream takes place, and further turbulences with shear stress arise, which shear stress causes further lyzing.

By impact on the housing this sludge stream changes its direction to the opposite one and is directed to the output openings of the outside partitions, through which openings the stream exits the disintegrator into the sludge space confined by the housing.

During the reverse streaming to the output openings the sludge impacts on the working surfaces of the rotating elements and lyzing takes place, wherein, the lyzing effect is increased by the surfaces of the output openings, where stream of sludge changes its direction to the axial direction into the sludge space, wherein the shear forces arising between the working surfaces of the rotating elements and the edges of openings of the outside partitions support further lyzing.

The rotating elements move with circumferential speed 60 - 100 m/s, preferably 80 - 90 m/s. This high speed and structure of the rotating elements cause further lyzing effect, and this by hydrodynamic cavitation because liquid -suspension is subjected to disintegration. The double working surface of the rotating element generates pressure on the face surfaces, which pressure changes to underpressure directly on the rear side of the rotating elements. A shock wave comes into existence having destructive effect on the surroundings, i.e. also on the microbial cells.

Arrangement of the disintegrator according to the invention is of simple design. This arrangement can be easily used in any centrifuge without any disassembly of the drum, only the housing of the end cylindrical part of the drum is extracted and modified by inserting of the static part of the disintegrator, what is one central and two outside partitions joined with the load-bearing ring. The static part of the disintegrator according to the invention is integrated into the housing, which housing consists of two halves fixed to the centrifuge frame. The rotating elements of the disintegrator according to the invention are suitably placed directly in the end part of the centrifuge drum in the axes of the exiting sludge stream.

By the disintegration of the thickened sludge according to the invention sufficient lyzing effect is reliably reached in this sludge, what is especially preferable because of an increase in its biodegradability, and thereby also to an increase in production of the biogas in the anaerobic stabilization of the sludge.

The disintegration has two effects. On the one hand, a decrease in the particle size of sludge is reached, and thereby the surface area of particles is increased, what has beneficial influence on the subsequent biological decomposition. On the other hand, the other disintegration - lyzing effect is destruction of the microorganism cells, what releases the cell content - lyzate to the surroundings, which lyzate contains enzymes and further biologically active compounds, that positively co-act in deeper decomposition of the organic compounds in anaerobic process. The resulting effect and objective of the lyzing is an increase in production of biogas and the with it related decrease in the quantity of organic compounds in the stabilized sludge. A side effect of the lyzing is a decrease in viscosity of the thickened lyzed sludge, what positively influences the degree of sludge thickening and its pumpability.

The lyzing effect is determined as an increase in the quotient of concentration of the dissolved organic compounds to the total concentration of organic compounds in the lyzed sludge. The organic compounds are determined as CHSK_{Cr}. By lyzing, 5% - 10% of organic compounds is converted to the liquid phase. This effect is reached by lyzing according to the invention at minimal increase in the electric power consumption, which consumption is in order of units of per cents of the total power input of the centrifuge. The share of the destroyed microbial cells is further doubled within 24 hours, because the damaged cells also decompose. Therefore, concentration of the organic compounds transferred into the solution is also doubled. The liquid phase is so enriched by 10 % - 20 % of the organic compounds. This process has another important positive operational effect, because by lyzing viscosity of the thickened sledge is so decreased that it is possible to concentrate it the dry matter content 12% by weight to 14% by weight, wherein it is reliably pumpable.

### Brief Description of the Drawings

Advantages and features of the presented invention will be explained by the description and references to the attached drawings, in which:
Fig. 1 shows total view of the disintegrator according to this invention in longitudinal section;
Fig. 2 shows position of one rotating element in the centrifuge drum according to the invention in section;
Fig. 3 shows position of one rotating element in drum between two output openings according to the invention in section;
Fig. 4 shows a view to a part of the central partition according to this invention;
Fig. 5 shows a view to a part of the outside partition according to this invention;
Fig. 6 shows a top plan view of the rotating element;
Fig. 7 shows position of the positioned rotating elements with regard to the output openings of the centrifuge drum according to the invention and als shows the plane of the exiting stream of sledge.

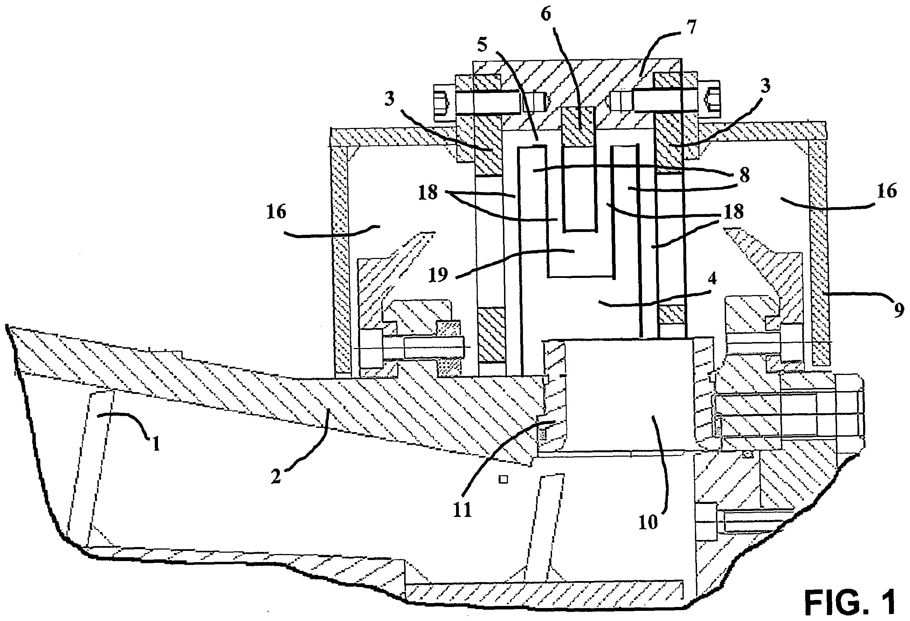

An example layout of a disintegrator according to this invention and its position in a thickening centrifuge is shown in Fig. 1. The disintegrator according to this invention is formed by a static part and a rotating part. By cooperation of these parts, lyzing of the thickened biological sludge takes place. The static part is put together from the static elements and forms a lyzing space **18,** in which space move the four-square rotating elements **4.** In the four-square rotating elements **4** a gap **19** is formed, into which gap **19** reaches the central partition **6.** The four-square rotating elements **4** are fastened to the centrifuge drum by screws so that a stream of the thickened sludge passes through the gap **19.** The static elements are adapted as a flat shaped annular area so that the two outside partitions **3** both surround the lyzing space **18** and allow to the lyzing sludge to exit this space. The central partition **6** divides the sludge stream. The four-square rotating elements **4** are provided with two arms **8,** between which arms **8** reaches the central partition **6.** The thickened sludge, which enters the lyzing space **18** and is kept in this space **18** for a moment before exiting this space **18,** is subjected to disintegration both by own kinetic energy and the rotating elements **4.**

The static part of the disintegrator is formed by a load-bearing ring **7,** which load-bearing ring **7** is the carrying part for the two outside partitions **3** and the central partition **6** and is built in the housing **9** of the centrifuge The two outside partitions **3** and the central partition **6** are firmly joined with the load-bearing ring **7.**

The thickening centrifuge drum is provided with a conical part **2,** which conical part **2** passes into a cylindrical part provided with output openings **10** for output of the thickened sludge. The thickened sludge in the centrifuge is transported into this terminal cylindrical part of the centrifuge drum by a screw conveyor **1.** The output openings **10** are provided with tubular inserts **11** of hard metal resisting to abrasion.

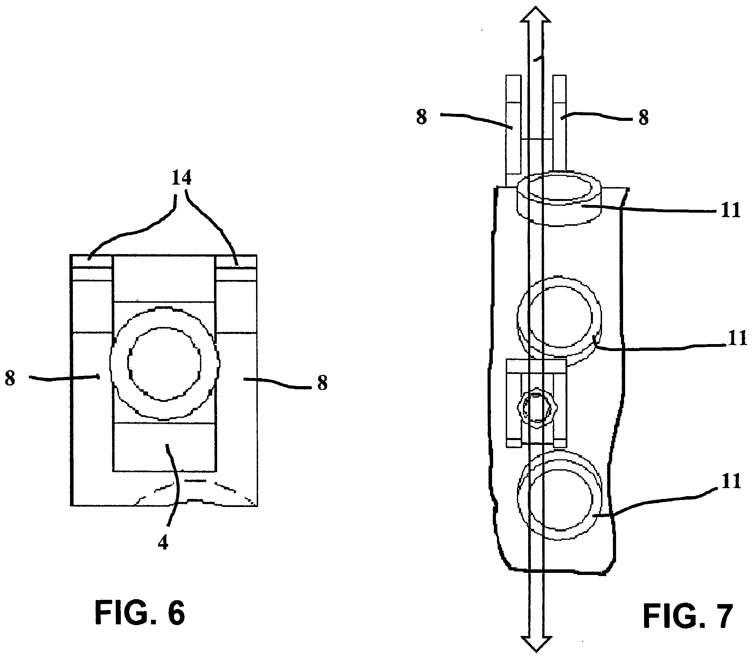

The sludge stream exiting from the tubular inserts **11** leaves the centrifuge drum radially in the form of a disc, which disc is indicated in Fig. 7 by a double arrow. Thickness of this disc is 1 - 2 cm. This sludge stream enters the lyzing space 18 and there it at first impacts on surfaces of the central partition **6,** the axial position of which is chosen so that the radially exiting sludge stream impacts on its centre. Thickness of the central partition **6** is 10 -15 mm. After impact on the central partition **6** the sludge stream moves on along it further in radial direction and impacts on the inner surface of the load-bearing ring **7,** where further destruction of cells in the sludge takes place by impact. The sludge cumulates in the space **5** delimited by the load-bearing ring **7,** the central partition **6,** and the two outside partitions **3.**

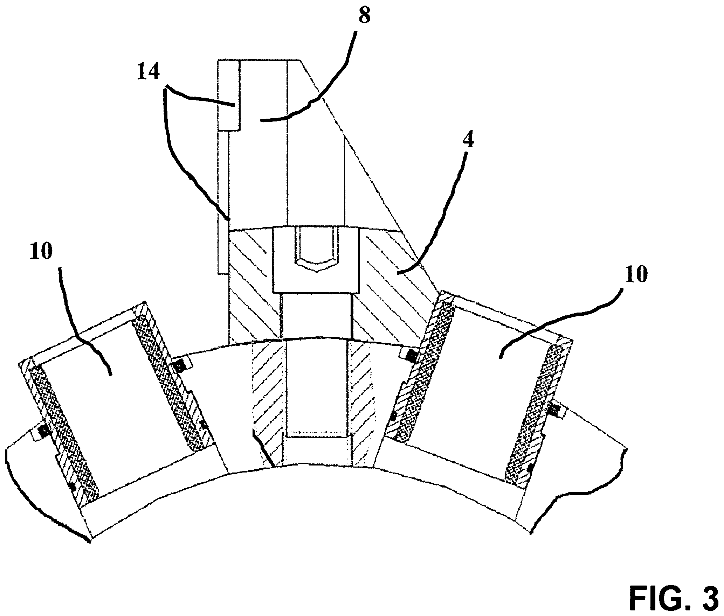

The face surfaces of the arms **8** of the four-square rotating elements **4** of the disintegrator, fastened to the cylindrical end part of the centrifuge drum so that they are placed between the tubular inserts **11** as shown in Fig. 3, reach into the sludge layer, which layer is concentrated in the space **5.** Number of the four-square rotating elements **4** is optional and the maximum number is determined by the number of gaps between the tubular inserts **11,** wherein their spacing has to be uniform to avoid disturbing of the centrifuge drum balance. The arranged four-square rotating elements **4** have to be of exactly the same weight. Fig. 3 also shows the form of the four-square rotating element **4,** when its rear part is bevelled to decrease weight of this element **4.** The face working surfaces of the arm **8** are preferably provided with plates **14** of sintered carbides.

The permanent sludge inflow into the space 5, which is closed, is a cause of the sludge revere flowing into the lyzing space **18.** From the lyzing space **18** the sludge flows through the openings **15** of both outside partitions **3,** which openings **15** have preferably trapezoidal shape, wherein here further destruction of cells takes place, because between the edges of the face surfaces of the arm **8** and the edges of the openings **15** in both outside partitions **3** the sludge is subjected to shear forces of the shear stress. Thereafter, the lyzed thickened sludge flows into the sludge space **16** of the centrifuge, and from there it flows out of the centrifuge for further use.

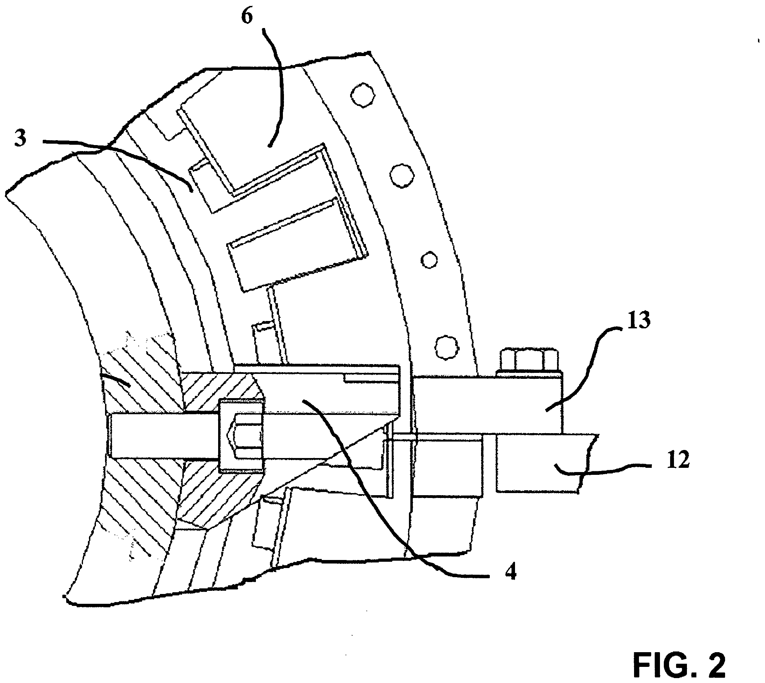

Fig. 2 shows fastening of the centrifuge housing **9,** which housing **9** is a part of it and is joined with the load-bearing ring **7** and two outside partitions **3** and the central partition **6** to the centrifuge frame **12** by means of a fastening footing **13** of the housing **9.** The centrifuge housing **9** with the inbuilt load-bearing ring **7** and two outside partitions 3 and a central partition **6** is divided to two halves and forms the whole of the static part of the equipment according to invention.

Fig. 3 shows position of the four-squared rotating element **4** between the output openings **10.** Preferably, the face surfaces of the four-squared rotating elements **4** are provided with plates **14** of materials resistant to abrasion, e.g. of sintered carbides.

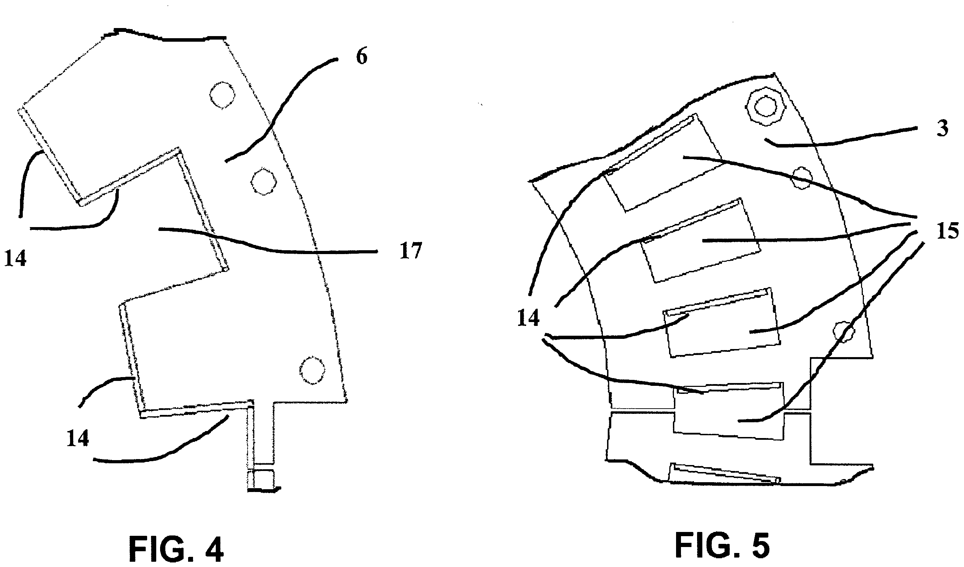

Fig. 4 shows form of the central partition **6** with a cut-out **17,** where preferably the cut-out surfaces can be provided with plates **14** of sintered carbides. The central partition **6** is made as an annular area divided into two halves.

Fig. 5 shows form of one of the outside partitions **3,** where preferably the working areas of the openings **15** can be provided with plates **14** of sintered carbides. The two outside partitions 3 are made as an annular area divided into two halves with a number of openings **15,** particularly in the form of an isosceles trapezoid.

Fig. 6 shows in top plan view a four-square rotating element 4 with arms 8 provided with plates **14** of hard metal on the face working surfaces.

The equipment for mechanical lyzing of the biological sludge according to the invention cannot in any case be the reason of centrifuge congestion, because any quantity of the thickened sludge, according to the inlet characteristics of the given centrifuge and the thickening set, can always flow through the openings **15** in the outside partitions **3,** wherein any volume of the thickened sludge flowing out of the equipment is effectively lyzed by impact, shear stress, and cavitation. In case of using the equipment according to this invention any mutual adjusting of the static rotating part also does not take place, and therefore, the repeated shut-downs of the centrifuge from operation, because design of this equipment allows continual effective mechanical lyzing of any quantity of the thickened sludge in accordance with the centrifuge capacity by the featurethat the radial stream of the thickened sludge always enters into the centre of the equipment, it is symmetrically divided, and both streams are subjected to the same destruction forces. The equipment is of simple design and uses a part of the centrifuges, which part is the independent housing of the terminal cylindrical part of the centrifuge drum to fasten the static parts of the equipment.

### Examples of Embodiments of the Invention

### Example 1

In use, efficacy of the invention is determined by determination of quantity of the organic compounds released by lyzing into the solution. The organic compounds are determined as CHSK_{Cr} in the liquid phase. Dry matter content of the input sludge into the thickening centrifuge was 1.1% by weight and concentration of the dissolved organic compounds determined as CHSK_{Cr} was 68 mg/l and after thickening to 6.1% by weight the concentration of the dissolved organic compounds determined as CHSK_{Cr} was 5350 mg/l. Concentration of the dissolved compounds was increased 78 fold.

### Example 2

Another effect of this invention consists in iscosity decreasing of the thickened lyzed sludge of Example 1, what is an important operational characteristic. The thickened sludge is pumped for further processing in the anaerobic fermentation and the sludge pumps are able to pump the thickened sludge with maximal dry matter content 6% by weight to 7% by weight, even if the thickening centrifuge is able to thicken to higher values up to the maximal load (what is its operational characteristic) of the given centrifuge by control of the difference in the rotation speeds. During thickening of sludge in the lyzing centrifuge it was determined that at the dry matter content in the thickened sludge 6% by weight viscosity was decreased (tangential stress) by 25%. Fluidity was increased, and at thickening to 11% by weight of the dry matter pumping of this sludge was without any problems.

### List of Reference Numerals

- 1: Screw conveyor
- 2: Conical part
- 3: Outside partition
- 4: Four-square rotating element
- 5: Space
- 6: Central partition
- 7: Load-bearing ring
- 8: Arm
- 9: Housing
- 10: Output opening
- 11: Tubular inserts
- 12: Frame
- 13: Footing
- 14: Plate
- 15: Opening
- 16: Sludge space
- 17: Cut-out
- 18: Lyzing space
- 19: Gap

## Claims

1. A centrifuge comprising an equipment for mechanical lyzing of biological sludge thickened in that centrifuge, which comprises rotating parts placed on a centrifuge drum and static parts joined firmly with a housing of the centrifuge, **characterized in that** the rotating parts are four-square rotating elements (4) symmetrically disposed between output openings (10) of the drum for the thickened sludge, wherein the static part consists of two outside partitions (3) and one central partition (6) in the shape of an annulus area, which partitions (3, 6) are arranged on a load-bearing ring (7) in the centrifuge housing (9) so that arms (8) of the four-square rotating elements (4) reach into the gaps between them.

2. Centrifuge according to claim 1, **characterized in that** the gap (19) into which the central partition (6) reaches, is formed in the four-square rotating elements (4).

3. Centrifuge according to claim 2, **characterized in that** the four-square rotating elements (4) are fastened to the centrifuge drum by screws so that a stream of the thickened sludge passes through the gap (19).

4. Centrifuge according to anyone of the claims 1 to 3, **characterized in that** the outside partitions (3) and the central partition (6) are formed in the shape of a divided annular area, wherein the outside partitions (3) are provided by openings (15) and the central partition (6) is provided with cut-outs (17).

## Patentansprüche

1. Zentrifuge, bestehend aus einer Einrichtung für mechanische Lyse des in diese Zentrifuge eingedichteten biologischen Schlamms, bestehend aus an der Trommel der Zentrifuge angeordneten Drehteilen und aus fest mit dem Deckel der Zentrifuge verbundenen statischen Teilen, **dadurch gekennzeichnet, dass** die drehenden Teile rechteckige, symmetrisch zwischen den für den eingedichteten Schlamm bestimmten Austrittsöffnungen (10) der Trommel angeordnete Drehelemente (4) sind, wobei der statische Teil aus zwei äußeren Trennwänden (3) und einem mittleren Trennwand (6) in der Form eines Kreisrings besteht, welche Trenwände (3,6) auf einem Tragring (7) im Deckel (9) der Zentrifuge so angeordnet sind, dass die Schenkel (8) der rechteckigen Drehelementen (4) in die Lücken zwischen sie ihnen gelangen.

2. Zentrifuge nach Anspruch 1, **dadurch gekennzeichnet, dass** in den rechteckigen Drehelementen (4) ein Spalt (19) gebildet ist, in den die mittlere Trennwand (6) eingreift.

3. Zentrifuge nach Anspruch 2, **dadurch gekennzeichnet, dass** die rechteckigen Drehelemente (4) zur Trommel der Zentrifuge durch Schrauben so befestigt sind, dass durch den Spalt (19) ein Strom des eingedichteten Schlamms läuft.

4. Zentrifuge nach mindestens einem der Ansprüche 1 bis 3, **dadurch gekennzeichnet, dass** die äußeren Trennwände (3) und die mittlere Trennwand (6) in der Form einer geteilten Ringfläche gebildet sind, wobei die äußeren Trennwände (3) mit Öffnungen (15) und die mittlere Trennwand (6) mit Ausschnitten (17) versehen sind.

## Revendications

1. Centrifugeuse comprenant un équipement pour la lyse mécanique d'une boue biologique épaissie dans cette centrifugeuse, qui comprend des parties rotatives placées sur un tambour de centrifugeuse et des parties statiques solidement reliées à un carter de la centrifugeuse, **caractérisée par le fait que** les parties rotatives sont des éléments rotatifs à quatre côtés (4) disposés de manière symétrique entre des ouvertures de sortie (10) du tambour pour la boue épaissie, la partie statique consistant en deux cloisons extérieures (3) et en une cloison centrale (6) sous la forme d'une zone annulaire, lesquelles cloisons (3, 6) sont agencées sur un anneau porteur (7) dans le carter de centrifugeuse (9) de telle sorte que des bras (8) des éléments rotatifs à quatre côtés (4) atteignent les intervalles entre elles.

2. Centrifugeuse selon la revendication 1, **caractérisée par le fait que** l'intervalle (19), dans lequel la cloison centrale (6) arrive, est formé dans les éléments rotatifs à quatre côtés (4),

3. Centrifugeuse selon la revendication 2, **caractérisée par le fait que** les éléments rotatifs à quatre côtés (4) sont fixés au tambour de centrifugeuse par des vis de telle sorte qu'un courant de boue épaissie passe par l'intervalle (19).

4. Centrifugeuse selon l'une quelconque des revendications 1 à 3, **caractérisée par le fait que** les cloisons extérieures (3) et la cloison centrale (6) sont sous la forme d'une zone annulaire divisée, les cloisons extérieures (3) comportant des ouvertures (15) et la cloison centrale (6) comportant des découpes (17).
